(19) Europäisches Patentamt
European Patent Office
Office européen des brevets

(11) Publication number: 0 005 300 A1

# (12) EUROPEAN PATENT APPLICATION

(21) Application number: 79200209.9

(22) Date of filing: 02.05.79

(51) Int. Cl.[2]: C 07 D 223/16, A 61 K 31/435 // C07C93/14

(30) Priority: 08.05.78 US 903623

(43) Date of publication of application: 14.11.79 Bulletin 79/23

(84) Designated Contracting States: AT BE CH DE FR GB IT NL SE

(71) Applicant: SCHERICO LTD., Töpferstrasse 5, CH-6004 Lucerne (CH)

(72) Inventor: Gold Elijah H., 10 Roosevelt Avenue, West Orange, New Jersey 07052 (US)
Inventor: Chang, Wei, 63 West Cedar Street, Livingston, New Jersey 07039 (US)

(74) Representative: Antony, Fritz, Dr. et al, P.O. Box 601 Winkelriedstrasse 35, CH-6002 Lucerne (CH)

(54) **Substituted 8-hydroxy-1-phenyl-2,3,4,5-tetrahydro-1H-3-benzazepines, process for the preparation thereof, and pharmaceutical compositions containing them.**

(57) Disclosed are novel 7-substituted 8-hydroxy-1-phenyl-2,3,4,5-tetrahydro-1H-3-benzazepines of the general formula I

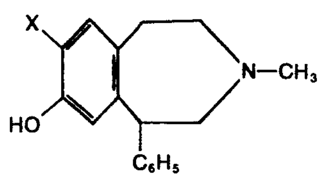

I

wherein X is chloro, bromo or trifluoromethyl, and the pharmaceutically acceptable acid addition salts thereof.

The novel compounds may be prepared by methods known per se. Preferably the compounds are prepared by dealkylation of a corresponding 8-alkoxy-1-phenyl-2,3,4,5-tetrahydro-1H-benzazepine at position 8.

The novel compounds are useful as intermediates in the preparation of corresponding esters and carbamates and show neuroleptic, antidepressive and antiagressive activity.

EP 0 005 300 A1

0005300

Substituted 8-hydroxy-1-phenyl-2,3,4,5-tetrahydro-1H-3-benzazepines, process for the preparation thereof, and pharmaceutical compositions containing them.

This invention relates to substituted 8-hydroxy-1-phenyl-2,3,4,5-tetrahydro-1H-3-benzazepines, processes for the preparation thereof, and pharmaceutical compositions containing them. The novel compounds of this invention show neuroleptic, antidipressive and antiagressive activity, and are also useful as intermediates in the preparation of corresponding esters and carbamates.

Substituted-1-phenyl-2,3,4,5-tetrahydro-1H-3-benzazepines have been previously described in the art, as for example, in U.S. Patent 3,393,192, British Patent 1,118,688, Danish Patent 123033, U.S. Patent 3,609,138 and U.S. Patent 4,011,319. The prior art has recognized that substituted—1-phenyl-2,3,4,5-tetrahydro-1H-3-benzazepines exhibit anti-bacterial effects, central nervous system effects and a hypotensive effect.

0005300

The novel compounds of this invention are the substituted 8-hydroxy-1-phenyl-2,3,4,5-tetrahydro-1H-3-benzazepines of the general formula I

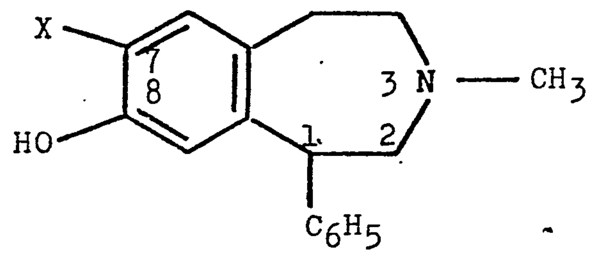

I

wherein X is chloro, bromo or trifluoromethyl, and the pharmaceutically acceptable acid addition salts thereof.

The group of compounds defined above by formula I is comprised by the definition of a class of 1-phenyl-2,3,4,5-tetrahydro-1H-3-benzazepines in the British Patent 1,118,688. However, neither the group of compounds defined above by formula I nor any specific member thereof is disclosed in said British patent.

Of the above compounds of formula I and their acid addition salts, the compounds wherein X is chloro or bromo, especially those wherein X is chloro are preferred. Of particular interest are the (S)-isomers of the compounds of formula I. (The compounds of formula I contain an assymmetric center at position 1).

The acid addition salts of the compounds of formula I can be derived from a variety of inorganic and organic acids such as for example sulfuric, phosphoric, hydrochloric, hydrobromic, hydroiodic, methanesulfonic, sulfamic, citric, lactic, pyruvic, oxalic, maleic, stearic, succinic, tartaric, fumaric, cinnamic, aspartic, acetic, benzoic, salicylic, gluconic, ascorbic and related acids.

The compounds of formula I can be prepared according to known methods:

A. by dealkylation of the corresponding 8-alkoxy compounds of the general formula II

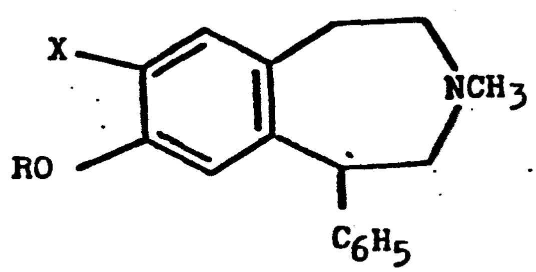

II

wherein R is alkyl and X is as defined for formula I. R is usually methyl but may also be any alkyl group containing e.g. up to 5 carbon atoms.

To cleave the alkoxy group and provide the hydroxy group at position 8, the compound of formula II may be reacted with a strong acid, such as for example aqueous hydrogen halide, preferably hydrogen bromide.

The reaction can advantageously be performed under a nitrogen atmosphere. The reaction is usually carried out in an aqueous medium (e.g. using 48 percent aqueous hydrobromic acid) at about 75 to 100°C, preferably at reflux temperature of the reaction mixture, for about 2 to 24 hours.

Other typical dealkylating procedures are the use of sodium ethanethiolate in an aprotic solvent such as dimethylformamide at 100-120°C for 4-6 hours, or the use of pyridine hydrochloride at 200-240°C for 1-2 hours.

Compound II may be prepared by cyclization of a compound of the general formula III

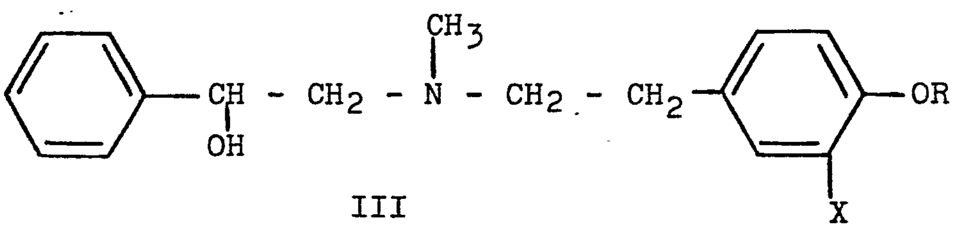

III

wherein X and R are as defined for formula II. The compound of formula III can be cyclized utilizing a dehydrating agent such as for example polyphosphoric acid, sulfuric acid, methane sulfonic acid, methane sulfonic acid/$P_2O_5$, zinc chloride and hydrogen fluoride. Especially preferred are sulfuric acid, methane sulfonic acid, methane sulfonic acid/$P_2O_5$, and hydrogen fluoride

maintained at about -10 to 20°C.

Convenient ways for preparing a compound of the general formula III can be summarized as follows:

1. reaction of a 2-phenyl-ethylamine with ethoxyethylbenzene

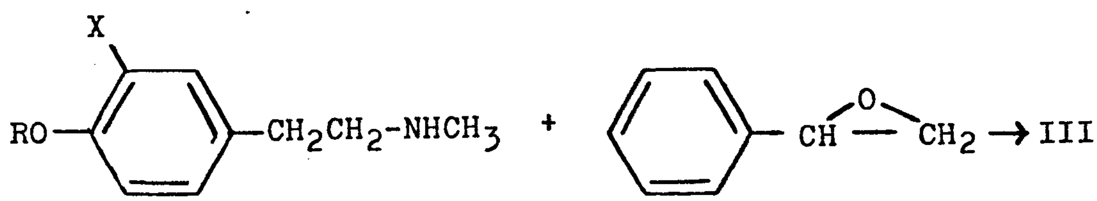

IV V

in a polar aprotic solvent such as acetonitrile, pyridine, dimethylformamide or dimethyl sulfoxide. The temperature at which the reaction is conducted is not critical, with room temperature to reflux being preferred.

2. reductive alkylation of compounds of formula VI, with aldehydes of formula VII, wherein R and X are as hereinbefore defined.

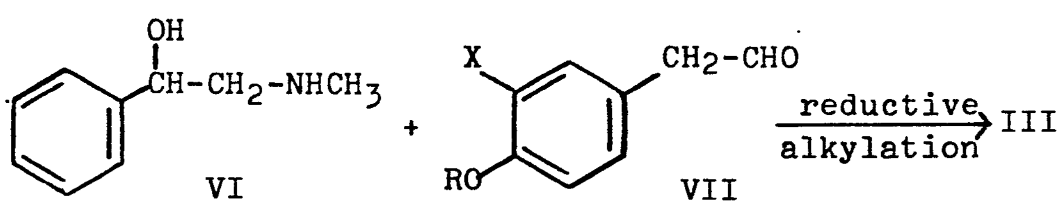


VI VII

This reaction is preferably carried out in a polar protic solvent such as 2-propanol or ethanol in the

presence of a reducing agent such as sodium borohydride, or in the presence of hydrogen and a catalyst such as palladium-on-carbon. The temperature at which the reaction is conducted is not critical, with room temperature to reflux being preferred.

3. reduction of compounds of formula VIII, wherein X and R are as hereinbefore defined. This reduction is preferably carried out in aprotic solvents, such as diethyl ether, tetrahydrofuran, dimethoxyethane, toluene, and the like, with a variety of reducing agents such as lithium aluminum hydride, diborane or sodium bis(2-methoxy) aluminum hydride. The temperature at which the reaction is conducted is not critical, with room temperature to reflux being preferred.

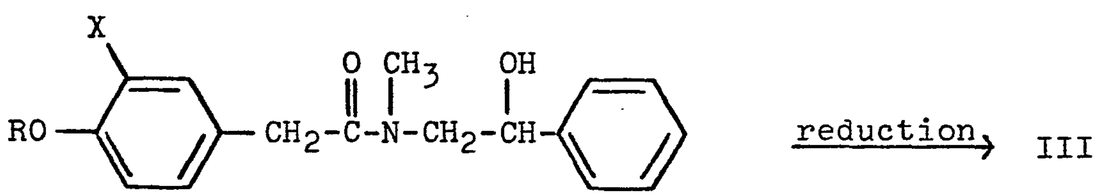


VIII

III

Compounds of formula VIII, wherein X and R are as hereinbefore defined, are readily prepared from compound VI and IX,

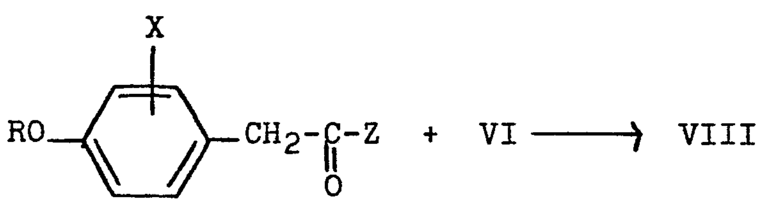

IX

VI

VIII

wherein X and R are as hereinbefore defined and Z is a leaving group such as halo, alkoxy, arylalkoxy, aryloxy, imidazolyl and the like, or is hydroxy. When Z is a leaving group the reaction is preferably carried out in a polar solvent such as water, pyridine, acetonitrile, tetrahydrofuran, dimethoxyethane or the like. The temperature at which the reaction is conducted is not critical, with room temperature to reflux being preferred. When Z is hydroxy, the reaction is preferably carried out in a polar aprotic solvent such as acetonitrile, tetrahydrofuran or dimethoxyethane in the presence of a dehydrating agent such as dicyclohexylcarbodiimide.

Compound II can also be prepared by introduction of the desired 7-substituent into an 8-alkoxy-1-phenyl-2,3,4,5-tetrahydro-1<u>H</u>-3-benzazepine containing no substituent or a replaceable substituent in position 7.

Compound II can also be prepared by N-methylation of a corresponding 8-alkoxy-1-phenyl-2,3,4,5-tetrahydro-1<u>H</u>-3-benzazepine

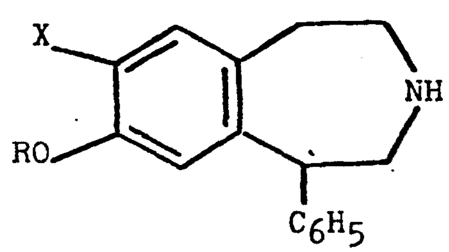

X

wherein R and X are as defined before. This N-methylation is accomplished in a conventional manner, a particularly facilitative method being the condensation of the compound of formula X with formaldehyde and formic acid. Alternatively, methyl chloride or methyl bromide may be used to methylate the compound. The compound of formula X can be prepared by known methods, for example analogously to the methods described above for the preparation of compound II.

B. by intramolecular cyclization of a compound of the general formula

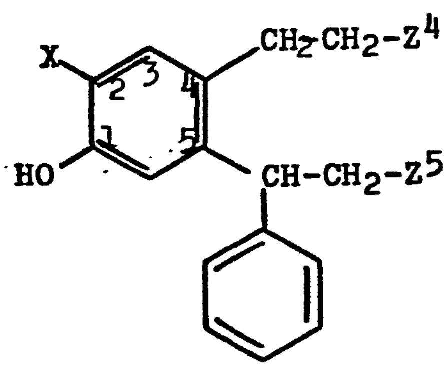

XI

wherein X is as defined for formula I and $Z^4$ and $Z^5$ are reactive groupings capable of forming the desired $>N-CH_3$ linkage under the reaction conditions employed. Preferably one of $Z^4$ and $Z^5$ is hydroxy or halogen and the other grouping is $-N{<}^{H}_{CH_3}$. When $Z^4$ or $Z^5$ is hydroxy the reaction is carried out by means of a dehydrating agent, such as for example polyphosphoric acid, sulfuric acid, methane sulfonic acid, methane sulfonic acid/$P_2O_5$, zinc chloride, hydrogen fluoride, hydrogen bromide, and sulfuric acid together with trifluoroacetic acid. Especially preferred are sulfuric acid, methane sulfonic acid, methane sulfonic acid/$P_2O_5$, and hydrogen fluoride

maintained at about -10 to +20°C. When $Z^4$ or $Z^5$ is halogen, preferably chloro or bromo, the compound of formula XI is for example reacted with $K_2CO_3$ in acetone, preferably at the reflux temperature of the reaction mixture. If desired, the hydroxy group at position 1 of the formula XI is protected before this process is carried out.

C. by hydrogenation of a 1-phenyl-3-benzazepine or 1-phenyl-dihydro-3-benzazepine of the general formula

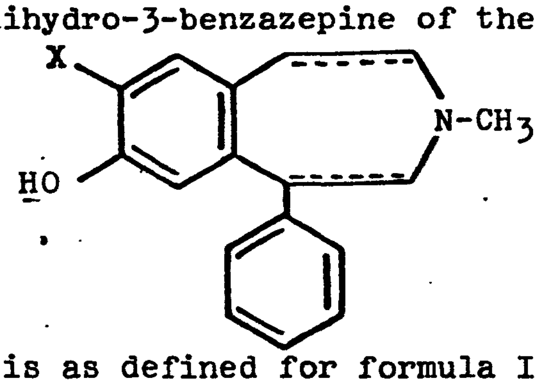

XII

wherein X is as defined for formula I and either one or both of the dotted lines represents an additional binding in the azepine ring. The compound of formula XII can be hydrogenated with catalytically activated or nascent hydrogen, e.g. by means of hydrogen over palladium. Preferably a compound of formula XII unsaturated in the 4,5-positions is used.

D. by methylation of an N-unsubstituted 1-phenyl-2,3,4,5-tetrahydro-1H-3-benzazepine of the general formula

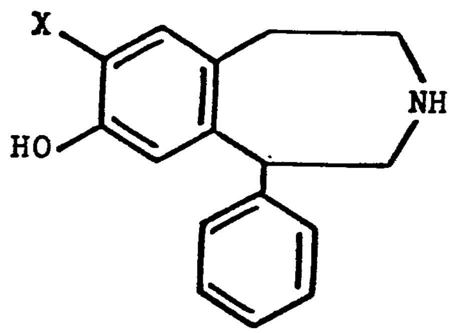

XIII

wherein X is as defined for formula I.

The compound of formula XIII can be methylated by condensation with a methylhalide (preferably bromide or chloride). Typically the reaction is effected in an inert solvent such as acetone, preferably at reflux temperature and in the presence of a basic condensing agent such as anhydrous potassium carbonate.

Alternatively the compound of formula XIII can be methylated using formaldehyde and formic acid in an inert solvent such as diethylether. Preferably 37% formaldehyde and 90% formic acid are used. Typically the reaction is carried out at reflux temperature.

E. by reducing a compound of the general formula

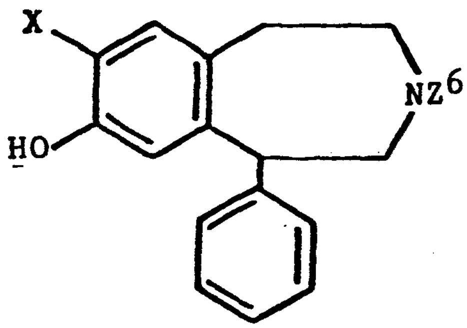

XIV

wherein X is as defined for formula I and $Z^6$ is a reducable group such as -CHO and $-CO_2R'$ (R' being alkyl). The compound of formula XIV can be reduced with diborane or with lithium aluminium hydride in an inert solvent such as tetrahydrofuran, dioxan or diethylether. The reaction is usually carried out at the reflux temperature of the reaction mixture.

F. by hydrolysis of 8-esters and/or 8-carbamates of the compounds of formula I. Many of these esters and carbamates are novel compounds, which are disclosed in the copending patent applications .

G. by hydrogenation of 1-phenyl-7-X-2,3,4,5-tetrahydro-1*H*-3-benzazepines, wherein X is as defined for formula I containing a protected hydroxy group in position 8. The protecting group is for example an aralkyl group such as $ArCH_2$- or $Ar_2CH$-, wherein Ar is aryl preferably phenyl.

The compounds of formula I, II, X, XII, XIII and XIV contain an asymmetric carbon atom in position 1 (chiral

center). By the above described processes racemic mixtures of the relevant (R)- and (S)- isomers are obtained, Separation of the isomers can be effected by the usual well known techniques. If an (R)- or (S)-isomer of the compound of formula II or X is used as starting material in the preparation of the desired compound I, the corresponding isomer of the compound I is obtained by the dealkylation described above.

The (R)- and (S)-isomers can be resolved by techniques such as for example resolution by means of forming an adduct (e.g. of urea), chromatography, crystallisation from optically active solvents, resolution via diastereoisomeric salts (e.g. by means of N-acetyl-D-leucine), resolution by enzymatic processes (including destruction and chemical transformation of one isomer) and destruction of one isomer. It is preferred to use fractional crystallization.

The compounds of formula I can be transformed into pharmaceutically acceptable acid addition salts by reaction with a pharmaceutically acceptable acid or a reactive derivative thereof. Also the free compounds of formula I can be set free from their acid addition salts.

The compounds of this invention are typically formulated into conventional pharmaceutical compositions according to accepted procedures. The dosage of these compounds is dependent upon various factors, such as the particular compound employed and the patient's individual response. Preferably, the compositions will contain the active ingredient in an active but non-toxic amount selected from about 0.8 mg to about 200 mg of active ingredient per dosage unit.

The pharmaceutical carrier employed in the formulation of such compositions may be a solid or liquid. Typical carriers include, but are not limited to: sugars such as lactose, sucrose, mannitol and sorbitol; starches such as tapioca starch and potato starch:, cellulose

derivatives such as sodium carboxymethylcellulose, ethyl cellulose and methyl cellulose, gelatin; calcium phosphates such as dicalcium phosphate and tricalcium phosphate; sodium sulfate; calcium sulfate, polyvinyl alcohols; stearic acid; alkaline earth metal stearates such as calcium stearate; stearic acid vegetable oils such as peanut oil, cottonseed oil, sesame oil, olive oil and corn oil; non-ionic, cationic and anionic surfactants; ethylene glycol polymers, β-cyclodextrin; fatty alcohols and hydrolyzed cereal solids; as well as other non-toxic compatible fillers, binders, disintegrants and lubricants commonly used in pharmaceutical formulations. For injectable solutions sterile water and other steril ingredients have to be used.

Antipsychotic drugs are widely used e.g. in the treatment of schizophrenia. Their use is generally limited by their propensity to cause neurologic, autonomic and/or endocrine side effects. The compounds of this invention are novel antipsychotic drugs. The compounds have effects common to standard antipsychotic drugs but may be regarded as having a more generally better biological profile. Representative compounds of the invention have been found to have specific antipsychotic, antiaggression and antidepressant activity with low potential for producing extrapyramidal, autonomic and hormonal side effects; the compounds did not show sedative and anti-convulsant

activity. On this basis the compounds of this invention are useful agents for the treatment of manifestations of psychotic disorders due to their combination of neuroleptic, antidepressant and antiaggressive properties. The compounds are thus indicated for reducing excitement, hypermotility, abnormal initiative, affective tension and agitation through their inhabitory effects on psychomotor functions of the treated subjects. In particular these compounds are indicated as useful in the treatment of schizophrenia, mental depression, anxiety and hyperactive agitated behavioral states, particularly in mentally retarded patients.

The compounds of the invention may be co-administered intravenously with a narcotic to produce neuroleptic analgesia. Such a state provides sufficient CNS action to permit certain procedures (e.g. bronchoscopy x-ray studies, burn dressing, cystoscopy) to be performed without additional medication. Addition of inhaled nitrous oxide to the neuroleptic-narcotic combination would provide analgesia sufficient for surgical operations.

The daily dosage of the active ingredient is dependent upon various factors, such as the particular compound employed, the condition for which the compound is administered and the patient's individual response. Typical dosages for use as a neuroleptic, antidepressant or antiaggressive agent, especially in the treatment of schizophrenia, mental depression, anxiety and hyperactive agitated behavioral states, particularly in mentally retarded patients, would vary from about 0.1 to 15, preferably 0.5 to 10 mg/kg per day divided in 3 or 4 doses, administered orally or parenterally.

The neuroleptic, antidepressant and antiaggressive properties of the compounds of this invention may be ascertained by testing in standardized assays such as are described in Barnett, et.al., Psychopharmacologia (Berl.) 36, 281-290 (1974). The properties of the compounds of this invention can be illustrated by the following test results: (S)-7-chloro-8-hydroxy-3-methyl-1-phenyl-2,3,4,5-tetrahydro-1H-3-benzazepine, , has an $ED_{50}$ of 0.35mg/kg in the anti-muricide assay and an $ED_{50}$ of 2.0 mg/kg in the methamphetamine reversal assay. For this compound the $LD_{50}$ (oral dose in mice) is greater than 300 mg/kg.

Test Descriptions

Anti-muricide assay: Male Long Evans rats weighing 225-250 g are housed 1 rat per cage, and killers (rats show an instinctive mouse killing behavior (muricide))

are selected initially after being deprived of food for approximately 66 hours. This is the only time the rats are deprived of food. Food, ad libitum, is avaiable on the cage floor since food bins are not practical because they afford a hiding place for mice. The rats are tested for muricidal behavior the following day and once a week thereafter; non-killers are always eliminated. A non-killer is a rat which fails to kill a mouse placed into his cage within 5 minutes. Drugs are tested in the afternoon of the day following this weekly check for persistance of the killing behavior. Since the rats are not deprived of food or water and rarely attempt to eat the mouse, this behavior is not motivated by hunger. Four weeks after the first test for killing, the rats are injected, i.p., with placebo and then tested 30/60 minutes after the injection. Rats that do not kill are eliminated. Only rats that consistantly kill mice placed in their home cages are used for drug testing. Every treatment-group is comprised of 5 rats. Each rat is presented with a CFI male albino mouse at 30 and 60 minutes after the intraperitoneal injection (1 ml/kg) of test drug. Blockade of muricidal behavior is determined by observing the number of surviving mice 5 minutes after being introduced into the rat cages. The result is expressed in $ED_{50}$ values, which means that at the given dosage 50% of the tested rats do not kill mice.

Methamphetamine reversal assay: Groups of 10 mice are aggregated in a 11 x 26 x 13 cm plastic chamber. Test drugs are given orally thirty minutes prior to an intraperitoneal administration of methamphetamine at 15 mg/kg. (This dose of methamphetamine is about 1.5 times the $LD_{50}$ under these conditions and usually kills at least 90 percent of the animals.) As a control perphenazine at 1 mg/kg is used.(this dose of perphenazine usually affords complete protection.) The number of deaths in each group are counted 4 hours after administration of methamphetamine.

The following Examples illustrate the preparation of the novel compounds of this invention.

Example 1

(*S*)-7-chloro-8-hydroxy-3-methyl-1-phenyl-2,3,4,5-tetrahydro-1*H*-3-benzazepine

A. Reflux for 16 hours, a solution of 21.0 g (0.113 moles) of 2-(3-chloro-4-methoxyphenyl)ethylamine and 13.6g (0.113 moles) of epoxyethylbenzene in 75 ml of acetonitrile. Remove the solvent and triturate the residue with 30 ml of ether. Filter and digest the precipitate twice with 60 ml of isopropyl ether and obtain N-[2-(3-chloro-4-methoxyphenyl)ethyl]-2-phenyl-2-hydroxyethylamine , m.p. 95-96°C.

B. Add in small portions, 12.0 g (0.0394 moles) of the product of step A to 60 ml of concentrated sulfuric acid with cooling (ice bath) and stirring so that the temperature of the reaction mixture stays at 10 ± 5°C. Stir at 10°C for 30 minutes, then for another hour at 25°C. Pour onto 500 g of ice, and then carefully add 100 ml of concentrated ammonium hydroxide, following this with the careful addition of 30 g of solid sodium hydroxide with cooling in an ice-bath, not allowing the temperature to rise above 30°C. Extract with 300 ml of benzene. Separate the benzene layer and dry over anhydrous sodium sulfate. Remove the solvent to obtain

(R,S)-7-chloro-8-methoxy-1-phenyl-2,3,4,5-tetrahydro-1H-3-benzazepine as a pale yellow syrup. Prepare the acid maleate in ethanol and recrystallize this crude salt from ethanol to obtain an analytically pure sample, m.p. 171-173°C.

C. Heat to reflux to dissolve a mixture of 9.60 g (0.033 moles) of the product of step B and 5.80 g (0.033 moles) of N-acetyl-D-leucine in 180 ml of acetonitrile. Cool slowly to room temperature. Filter and recrystallize the salt repeatedly from acetonitrile to obtain (S)-7-chloro-8-methoxy-1-phenyl-2,3,4,5-tetrahydro-1H-3-benzazepine N-acetyl-D-leucinate, m.p. 171-173°C $[\alpha]_D^{26}$ = + 27.7° (c=1, ethanol). Stir 1 g of this salt in a mixture of 20 ml of 0.5 N sodium hydroxide and 20 ml of ether to total dissolution. Separate the ether layer, dry over anhydrous sodium sulfate, and evaporate to obtain (S)-7-chloro-8-methoxy-1-phenyl-2,3,4,5-tetrahydro-1H-3-benzazepine as a colorless solid. Recrystallize from ethyl ether to obtain an analytically pure sample, m.p. 81-82°C, $[\alpha]_D^{26}$ = +38.3° (c=1,ethanol).

D. Reflux for 4 hours, a solution of 52.0 g (0.18 moles) of the product of step C in a mixture of 96 ml of 37% formaldehyde and 144 ml of 90% formic acid. Distill almost to dryness at 100°C under reduced pressure

(about 100 ml). Dissolve the residue in a mixture of 500 ml of 1 N sodium hydroxide and 500 ml of ethyl ether with cooling and stirring. Separate the ether layer, dry over anhydrous sodium sulfate, and evaporate to give (S)-7-chloro-8-methoxy-3-methyl-1-phenyl-2,3,4,5-tetrahydro-1H-3-benzazepine as a colorless solid. Obtain an analytically pure sample by crystallization from ethyl ether, m.p. 91-92°C, $[\alpha]_D^{26}$ = +47.3° (c=1, ethanol).

E. Heat and stir a mixture of 0.850 g (0.0028 moles) of the product of step D in 12 ml of 48% hydrobromic acid at 100°C for 16 hours. Dilute with 75 ml of water and heat on a steam bath to dissolve. Adjust to about pH 8 with solid sodium bicarbonate, filter, and wash the resulting precipitate with water. Recrystallize from ethanol to obtain analytically pure (S)-7-chloro-8-hydroxy-3-methyl-1-phenyl-2,3,4,5-tetrahydro-1H-3-benzazepine, m.p. 188-189°C, $[\alpha]_D^{26}$ = +44.8° (c=1,dimethylformamide).

## Example 2

(S)-7-bromo-8-hydroxy-3-methyl-1-phenyl-2,3,4,5-tetrahydro-1H-3-benzazepine

A. Hydrogenate a solution of 21.10 g (0.07 moles) of (S)-7-chloro-8-methoxy-3-methyl-1-phenyl-2,3,4,5-tetrahydro-1-H-3-benzazepine (e.g. obtained as described in Example 1, step D) 3.30 g (0.0825 moles) of sodium hydroxide in 300 ml of 95% ethanol at room temperature and at 4.2 $kg/cm^2$ (=60 psi),with 1.70 g of 20% palladium hydroxide-on-carbon until the theoretical amount of hydrogen is consumed. Remove the catalyst and concentrate the filtrate to dryness on a steam bath at about 100 mm. Stir the residue with a mixture of 100 ml of water and 150 ml of ethyl ether to total dissolution. Separate the ether layer, dry over anhydrous sodium sulfate, and evaporate to dryness. Crystallize the residue from hexane to obtain analytically pure (S)-8-methoxy-3-methyl-1-phenyl-2,3,4,5-tetrahydro-1H-3-benzazepine, m.p. 50-51°C, $[\alpha]_D^{26}$ = +51.8° (c=1, ethanol).

B. Warm a solution of 6.17 g (0.023 moles) of the product of step A in 46 ml of glacial acetic acid (containing 3.0 g of anhydrous hydrobromic acid) and add

dropwise a solution of 33.5 ml of bromine in glacial acetic acid (0.186 g/ml) with stirring. Stir at room temperature for 2.5 hours after the addition is complete and then for 5 minutes at 100°C. Pour the reaction mixture into 600 ml of ice, basify with excess 50% sodium hydroxide, and extract with 200 ml of ethyl ether. Separate the ether layer and dry over anhydrous sodium sulfate. Precipitate (S)-7-bromo-8-methoxy-3-methyl-1-phenyl-2,3,4,5-tetrahydro-1H-3-benzazepine hydrochloride with 1N ethereal hydrogen chloride as a gummy solid. Crystallize from ethanol, to obtain a colorless solid, m.p. 263-265°C.

C. Heat for 16 hours at 115°C a mixture of 2.04 g (0.0059 moles) of the product of step B in 20 ml of 48% hydrobromic acid. Pour the reaction mixture into 180 ml of boiling water and adjust the pH to about 8 with solid sodium bicarbonate. Cool to room temperature and extract with 150 ml chloroform. Separate the chloroform layer, dry over anhydrous sodium sulfate, and evaporate to give a viscous gum. Purify through a silica gel column (50 g thin layer chromatography grade), with chloroform:ethanol:ammonium hydroxide (90:2:0.5) as eluent, to obtain (S)-7-bromo-8-hydroxy-3-methyl-1-phenyl-2,3,4,5-tetrahydro-1H-3-benzazepine as a colorless syrup. Prepare the acid maleate and crystallize from ethanol,

m.p. 197-199°C, $[\alpha]_D^{26}$ = -0.7° (c=1, dimethylformamide).

Also other compounds of this invention can be prepared accordingly to the above examples, such as for example (R)-7-chloro-8-hydroxy-3-methyl-1-phenyl-2,3,4,5-tetrahydro-1H-3-benzazepine and its racemate.

The following formulations illustrate some of the dosage forms in which the compounds of this invention may be employed (Compound A mentioned in the formulation examples is (S)-7-chloro-8-hydroxy-3-methyl-1-phenyl-2,3,4,5-tetrahydro-1H-3-benzazepine maleate.)

Formulation 1

Enteric Coated Tablets

| | mg/core |
|---|---|
| Compound A | 100.0 |
| Citric Acid | 1.0 |
| Lactose, USP | 33.5 |
| Dicalcium Phosphate | 70.0 |
| Nonionic Surfactant (*Pluronic F-68) | 30.0 |
| Sodium Lauryl Sulfate | 15.0 |
| Polyvinylpyrrolidine | 15.0 |
| Polyethylene Glycol (*Carbowax 1500) | 4.5 |
| Polyethylene Glycol (Carbowax 6000) | 45.0 |
| Denatured Alcohol 50 ml/1000 cores | |
| Corn Starch | 30.0 |
| Dry | |
| Sodium Lauryl Sulfate | 3.0 |
| Magnesium Stearate | 3.0 |
| Tablet Weight | 350.0 |

[*Pluronic and Carbowax are Registered Trade Marks]

Procedure

Compound A is mixed with the citric acid, lactose, dicalcium phosphate, the pluronic and sodium lauryl sulfate. The above mixture is screened through a No.60

screen and damp granulated with an alcoholic solution consisting of polyvinylpyrrolidone, carbowax 1500 and 6000. Add additional alcohol, if necessary, to bring powders to a pasty mass. Add corn starch and continue mixing until uniform granules are formed. Pass through a No. 10 screen, tray and dry in an oven at 100°C for 12-14 hours. Reduce dried granulation through a No. 16 screen, add sodium lauryl sulfate and magnesium stearate, mix and compress into desired shape on a tablet machine.

Coating

The above cores are treated with a lacquer and dusted with talc to prevent moisture adsorption. Sub-coat layers are added to round out the core. A sufficient number of lacquer coats are applied to make the core enteric. Additional sub-coats and smoothing coats are applied to completely round out and smooth the tablet. Color coats are applied until desired shade is obtained. After drying the coated tablets are polished to give the tablets an even gloss.

Formulation 2

Capsules

| | mg/capsule |
|---|---|
| Compound A | 100.00 |
| Citric Acid | 1.00 |
| Nonionic Surfactant (Pluronic,F-68) | 40.00 |
| Sodium Lauryl Sulfate | 20.00 |
| Lactose | 238.00 |
| Magnesium Stearate | 1.00 |
| | 400.00 |

Procedure

Mix together Compound A, citric acid, pluronic, sodium lauryl sulfate and lactose. Pass through a No. 80 screen. Add magnesium stearate, mix and encapsulate into two-piece gelatin capsules of an appropriate size.

Formulation 3

Suppository

| | mg/2gms |
|---|---|
| Compound A | 100 |
| Theobroma Oil, Pharm. Grade | |
| to make | 2 gms |

Procedure

Prepare a slurry of the Compound A with a portion of the melted theobroma oil and pass the slurry through a

suitable colloid mill until it is free of grittiness. Add sufficient melted theobroma oil to bring the batch to final weight. Pour the melted mix, while maintaining uniformity, into appropriately prepared molds and allow to cool.

Claims

1. Substituted 8-hydroxy-1-phenyl-2,3,4,5-tetrahydro-1H-3-benzazepines of the general formula I

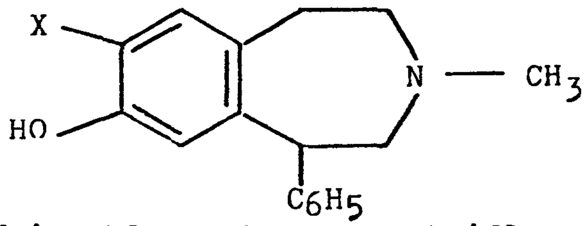

I

wherein X is chloro, bromo or trifluoromethyl, and the pharmaceutically acceptable acid addition salts thereof.

2. A compound according to claim 1, wherein X is chloro or bromo.

3. A compound according to claim 1 or 2 which has the (S)-stereochemical configuration at position 1, said isomer being substantially free of its (R)-isomer.

4. A compound according to claim 3, which is (S)-7-chloro-8-hydroxy-3-methyl-1-phenyl-2,3,4,5-tetrahydro-1H-3-benzazepine.

5. A compound according to claim 3, which is (S)-7-bromo-8-hydroxy-3-methyl-1-phenyl-2,3,4,5-tetrahydro-1H-3-benzazepine.

6. Process for the preparation of a compound of the general formula I and a pharmaceutically acceptable acid addition salt thereof, as defined in any one of claims 1 to 5, characterized in that

a) a corresponding 8-alkoxy-1-phenyl-2,3,4,5-tetrahydro-1H-3-benzazepine is dealkylated at position 8;

b) a compound of the general formula

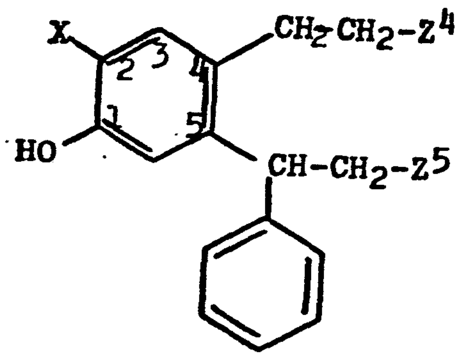

XI

wherein X is as defined for formula I and $Z^4$ and $Z^5$ are reactive groupings capable of forming the desired $>N-CH_3$ linkage under the reaction conditions employed is subjected to intramolecular cyclization;

c) a 1-phenyl-3-benzazepine or 1-phenyl-dihydro-3-benzazepine of the general formula

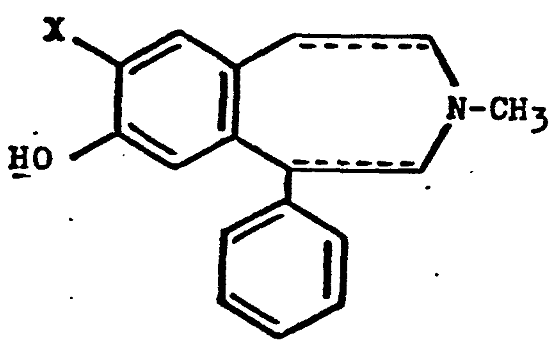

XII

wherein X is as defined for formula I and either one or both of the dotted lines represents an additional binding in the azepine ring is hydrogenated;

d) an N-unsubstituted 1-phenyl-2,3,4,5-tetrahydro-1H-3-benzazepine of the general formula

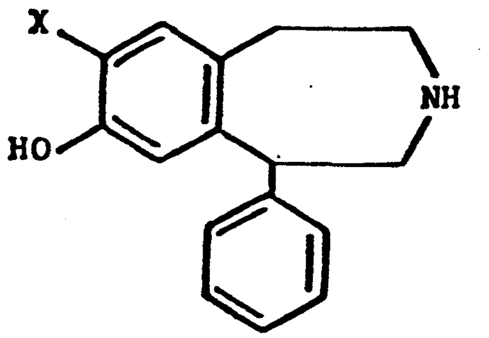

XIII

wherein X is as defined for formula I is N-methylated;

e) a compound of the general formula

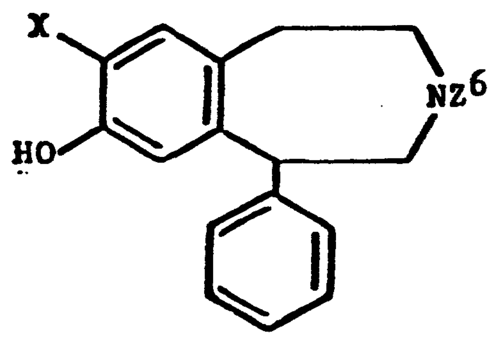

XIV

wherein X is as defined for formula I and $Z^6$ is a reducable group such as -CHO and $-CO_2R'$ (R' being alkyl) is reduced;

f) an 8-ester or an 8-carbamate of a compound of formula I is subjected to hydrolysis;

g) a 1-phenyl-7-X-2,3,4,5-tetrahydro-1H-3-benzazepine, wherein X is as defined for formula I, containing a protected hydroxy group in position 8 is subjected to hydrogenation;

and, if desired, the (R)- and/or (S)-isomer is isolated from a so obtained racemate and/or the resulting product is transformed into its pharmaceutically acceptable acid

addition salt and/or the free compound is set free from its acid addition salt.

7. Process according to claim 6a, characterized in that an 8-methoxy-1-phenyl-2,3,4,5-tetrahydro-1H-3-benzazepine is demethylated by means of a strong acid.

8. A therapeutic composition comprising as active ingredient one or more of the compounds as defined in any one of claims 1 to 5 or as produced by the process of claim 6 or 7.

European Patent Office

**EUROPEAN SEARCH REPORT**

0005300

Application number
EP 79 20 0209

**DOCUMENTS CONSIDERED TO BE RELEVANT**

| Category | Citation of document with indication, where appropriate, of relevant passages | Relevant to claim |
|---|---|---|
| D | GB - A - 1 118 688 (SCHERICO) * claims; page 6, lines 5-19; example 9 * --- | 1-2,4-8 |
| | GB - A - 1 268 243 (WALLACE & TIERNAN) * claims; page 2, line 18 - page 4, line 5; page 7, lines 1-14; page 32, lines 1-5; page 34, line 42 to page 35 * --- | 1-2,8 |
| | LU - A - 78 513 (SMITHKLINE) * claims; page 3, line 34 to page 4, line 2; page 4, line 3 to page 6, line 34 * --------- | 1-3,6,8 |

CLASSIFICATION OF THE APPLICATION (Int. Cl.[3])

C 07 D 223/16
A 61 K 31/435
C 07 C 93/14

TECHNICAL FIELDS SEARCHED (Int.Cl.[3])

C 07 D 223/16
A 61 K 31/435

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 10-07-1979 | NUYTS |

EPO Form 1503.1 06.78